# FASCICULE DE BREVET EUROPEEN

(11) **EP 0 476 118 B1**
(45) Date de publication et mention de la délivrance du brevet: **09.07.1997**
(21) Numéro de dépôt: 91907695.0
(22) Date de dépôt: 05.04.1991
(51) Int. Cl.: A61F 7/12, A61M 25/00, A61N 5/04

(54) **SONDE RECTALE**
REKTALSONDE
RECTAL PROBE

(30) Priorité: 06.04.1990 FR 9004442
(43) Date de publication de la demande: 25.03.1992
(73) Titulaire: TECHNOMED MEDICAL SYSTEMS, 69120 Vaulx-en-Velin (FR)
(72) Inventeur: CATHAUD, Muriel, F-69780 Vénissieux (FR); DEVONEC, Marian, F-01700 Miribel (FR)
(74) Mandataire: Portal, Gérard
(86) Numéro de dépôt international: FR9100279
(87) Numéro de publication internationale: WO9115174

(56) Documents cités:
- EP-A- 0 014 125
- EP-A- 0 091 405
- DE-C- 321 088
- FR-A- 608 139
- FR-A- 1 304 740

## Description

La présente invention concerne essentiellement une sonde rectale. Plus généralement, la présente invention concerne un dispositif formant sonde rectale, ainsi qu'un appareil de traitement thérapeutique, notamment pour le traitement thérapeutique de la prostate, comportant un tel dispositif sonde rectale. Cette sonde rectale est particulièrement appropriée pour réaliser un traitement thérapeutique de tissus par hyperthermie, et en particulier de la prostate.

Dans la technique antérieure, on connaît par le document EP-A-0 248 758, un applicateur à micro-ondes inséré dans le rectum pour le traitement de la prostate par hyperthermie. L'applicateur à micro-ondes est bloqué dans le rectum par un ballonnet latéral.

Un blocage de l'applicateur dans le rectum par l'intermédiaire d'un ballonnet latéral présente l'inconvénient majeur de déformer la paroi rectale et de la comprimer, ce qui aboutit à une réduction significative de la circulation sanguine. Or, la circulation sanguine est le principal facteur d'évacuation des calories déposées dans la paroi rectale par les micro-ondes. La compression de la paroi rectale peut donc entraîner une surchauffe importante et des risques de brûlures.

D'autre part, il est connu par le document DE-321 088 un dispositif de traitement des hémorroïdes comprenant une tête élastique de dimension considérablement plus grande que l'ouverture élargie du rectum, de sorte qu'après introduction de la tête, celle-ci applique une pression latérale suffisante pour comprimer de manière sensiblement uniforme les hémorroïdes jusqu'à disparition (page 1, lignes 42 à 52).

Par ailleurs, il est connu par le livre de Xavier Bender intitulé la Haute-Fréquence en Gynécologie, éditeur l'Expansion Scientifique Française, 23 rue du Cherche-Midi, Paris (1933), pages 222 à 226, divers dispositifs de diathermie rectale, de type rigide qui constituent des dispositifs de traitement thérapeutique thermo-électrique par voie rectale.

Encore, le brevet français N°922.239 (Cagnard) et son addition N°57.824 décrivent des électrodes rectales utilisées en électrothérapie qui constituent des dispositifs de traitement thérapeutique thermo-électrique par voie rectale.

Le document EP-A-0 144 125 est relatif à un cathéter urétral pour drainer la vessie comprenant un capteur de température sous forme d'un fil hélicoîdal. Une application rectale n'est pas envisagée ni suggérée.

La présente invention a donc pour but de résoudre le nouveau problème technique consistant en la fourniture d'une solution permettant de positionner un dispositif quelconque de détection dans le rectum, sans pour autant réaliser un effet de compression sur le rectum. De préférence, cette solution doit également permettre d'épouser la forme du rectum.

La présente invention a encore pour but de résoudre le nouveau problème technique consistant en la fourniture d'une solution permettant de mesurer la température dans le rectum de façon simple, fiable, ce qui est particulièrement utile lors d'un traitement thérapeutique d'un organe disposé au voisinage du rectum, en particulier de la prostate, notamment par hyperthermie.

Ces problèmes techniques doivent également être résolus d'une manière particulièrement simple, aisée à mettre en oeuvre, peu coûteuse, et donc utilisable à l'échelle industrielle.

Les problèmes techniques énoncés ci-dessus et ainsi que d'autres qui seront apparents à l'homme de l'art sont résolus par la présente invention.

Ainsi, selon un premier aspect, la présente invention fournit un dispositif formant sonde rectale, caractérisé en ce qu'il comprend un corps de sonde réalisé en un matériau polymère autoportant souple dont je degré de souplesse est défini par la dureté Shore A, qui est inférieure à environ 90, pour épouser la forme du rectum (R), tout en ne réalisant sensiblement pas d'effet de compression sur le rectum (R), lorsqu'il est introduit dans le rectum ; et en ce que le corps de sonde (12) comprend :
a) des moyens de mesure d'un champ électrique, ou
b) au moins un dispositif capteur de température, les moyens de mesure ou le dispositif étant disposés en combinaison avec un moyen d'arrêt (16) pour faciliter le positionnement du dispositif capteur de température ou des moyens de mesure d'un champ éléctrique en regard d'un organe à observer ou à traiter, en particulier en regard de la prostate.

Selon un mode de réalisation avantageux du dispositif formant sonde rectale selon l'invention, le matériau polymère autoportant souple précité a une dureté Shore A comprise entre 20 et 90, en particulier entre 60 et 80.

Selon un autre mode de réalisation avantageux du dispositif formant sonde rectale selon l'invention, celui-ci est caractérisé en ce que le corps de sonde précité comprend un rétrécissement de diamètre en regard du sphincter, ce rétrécissement favorisant un maintien de la sonde en place par le sphincter, de préférence la longueur du rétrécissement correspond sensiblement à la dimension du sphincter.

Selon un autre mode de réalisation avantageux de l'invention, l'épaisseur du corps de sonde varie entre environ 1 et environ 3,5 mm selon la dimension.

Selon un autre mode de réalisation avantageux de l'invention, le corps de sonde est réalisé en un matériau polymère présentant une dureté Shore A comprise entre 60 et 80, et est de préférence choisi parmi un élastomère tel qu'un caoutchouc, un polyuréthanne ; ou une matière plastique telle qu'un polyéthylène ou du PVC.

Avantageusement, le corps de sonde précité est pourvu d'un moyen d'arrêt d'insertion de la sonde dans le rectum.

Selon un mode de réalisation préféré, ce moyen d'arrêt d'insertion comprend une languette s'étendant extérieurement radialement par rapport au corps de la sonde, de préférence dans un plan sensiblement perpendiculaire au corps de la sonde, encore de préférence réalisée avec le même matériau que le corps de sonde.

Selon encore un mode de réalisation avantageux, la longueur du corps de la sonde rectale permet d'aller au moins en regard d'un organe à observer, à ou traiter, en particulier de la prostate, en position insérée dans le rectum pour permettre de capter la température par le dispositif capteur de température précité, ou de mesurer un champ électrique par les moyens de mesure d'un champ électrique précité.

Selon une autre variante de réalisation avantageuse, le corps de la sonde précité est creux, tout en étant sensiblement fermé à son extrémité avant, et ouvert à son extrémité arrière ; avantageusement, il comprend une ouverture permettant l'insertion d'un instrument de détection ou de repérage d'un organe à traiter, en particulier de la prostate, de préférence comprenant une sonde échographique, en particulier de type ultrasonique permettant de visualiser l'organe à traiter, en particulier la prostate. De préférence, ce dispositif capteur de température ou les moyens de mesure de champ électrique est disposé du même côté que la languette précitée de façon à faciliter le positionnement des capteurs de température ou des moyens de mesure en regard d'un organe à observer, en particulier la prostate.

Selon encore une caractéristique particulière du dispositif formant sonde rectale de l'invention, le corps de sonde creux précité comprend un orifice à son extrémité avant interne, permettant le passage des gaz éventuellement présents dans le rectum.

Selon une réalisation particulièrement avantageuse, le capteur de température précité comprend au moins un thermomètre à fibre(s) optique(s) logée(s) dans une rainure de la paroi du corps de la sonde, en particulier dans une rainure interne à la paroi du corps de la sonde.

De préférence, on peut prévoir l'emploi de plusieurs thermomètres à fibres optiques disposées décalées radialement et/ou longitudinalement dans le sens longitudinal de la sonde, de manière à détecter la température sur diverses positions radiales et/ou longitudinales de la sonde.

Selon un deuxième aspect, la présente invention concerne aussi un appareil de traitement thérapeutique, caractérisé en ce qu'il comprend un dispositif formant sonde rectale tel que précédemment défini.

Avantageusement, cet appareil de traitement thérapeutique comprend des moyens de traitement thérapeutique de tissus par hyperthermie, de préférence de traitement thérapeutique de la prostate. Dans ce cas, la sonde rectale reçoit de préférence un instrument de détection ou de repérage de l'organe à traiter, en particulier de la prostate. De préférence, cet instrument de détection comprend une sonde échographique, en particulier de type ultrasonique permettant de visualiser l'organe à traiter, en particulier la prostate.

Selon un mode de réalisation avantageux, les moyens de traitement thérapeutique de tissus comprennent un dispositif générateur de champ électrique introduit dans l'urètre U jusque dans la prostate pour réaliser un traitement thérapeutique de la prostate, par exemple comprenant une sonde émettrice à micro-ondes comprenant un ballonnet de positionnement introduit jusque dans la vessie.

On conçoit ainsi qu'avec la présente invention, on résout les problèmes techniques précédemment énoncés et on obtient les avantages techniques déterminants précédemment énoncés, ainsi que ceux qui sont apparents à l'homme de l'art.

D'autres buts, caractéristiques et avantages de l'invention apparaîtront clairement à la lumière de la description explicative qui va suivre faite en référence aux dessins annexés représentant un mode de réalisation actuellement préféré de l'invention, donné simplement à titre d'illustration et qui ne saurait donc en aucun façon limiter la portée de l'invention. Dans les dessins :
- la figure 1 représente une vue schématique en coupe partielle d'un mode de réalisation actuellement préféré d'un dispositif formant sonde rectale selon l'invention, en position insérée dans le rectum ;
- la figure 2 représente une vue en coupe axiale longitudinale, agrandie du dispositif formant sonde rectale de la figure 1 ; et
- la figure 3 représente une vue de face selon la flèche III de la figure 2.

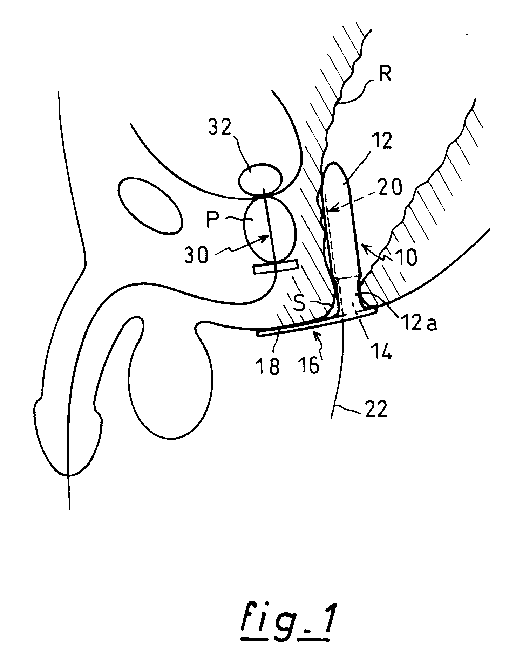

En référence aux figures 1 à 3, un dispositif formant sonde rectale selon la présente invention est représenté par le numéro de référence général 10. Ce dispositif formant sonde rectale 10 est caractérisé en ce qu'il comprend un corps de sonde 12. De préférence, ce corps de sonde est creux, c est-à-dire comporte une cavité interne 13 qui n'est pas complètement traversante de sorte que l'extrémité avant 12b est fermée. Elle peut, cependant, comporter un orifice 26 permettant l'évacuation des gaz éventuellement contenus dans le rectum. Ce corps de sonde 12 est réalisé en un matériau polymère autoportant souple, dont le degré de souplesse est déterminé pour permettre d'épouser la former du rectum R, comme cela est clairement visible à la figure 1, tout en ne réalisant sensiblement pas d'effet de compression sur le rectum, ce qui est encore clairement visible à la figure 1, lorsqu'il est introduit dans le rectum.

De préférence, ce degré de souplesse est défini par la dureté Shore A qui est inférieure à environ 90.

Encore de préférence, le matériau polymère a une dureté Shore A qui est comprise entre 20 et 90, en particulier entre 60 et 80. Des exemples du matériau polymère présentant une telle dureté Shore A entre 60 et 80 sont un élastomère tel qu'un caoutchouc, un polyuréthanne ; ou une matière plastique telle que le polyéthylène ou du PVC. L'épaisseur du corps 12 peut varier entre environ 1 et environ 3,5 mm selon la dimension.

Selon un mode de réalisation actuellement préféré du dispositif formant sonde rectale 10, le corps de sonde 12 comprend un rétrécissement de diamètre 12a en regard du sphincter S, ce rétrécissement 12a favorisant un maintien de la sonde 10 en place par le sphincter. De préférence, la longueur 1 du rétrécissement 12a correspond sensiblement à la dimension du sphincter S, ce qui est encore visible à la figure 1.

Le corps de sonde 12 comprend à son extrémité arrière définie par le rétrécissement 12a, une ouverture 14 permettant l'insertion d'un instrument de détection ou de repérage de l'organe à traiter.

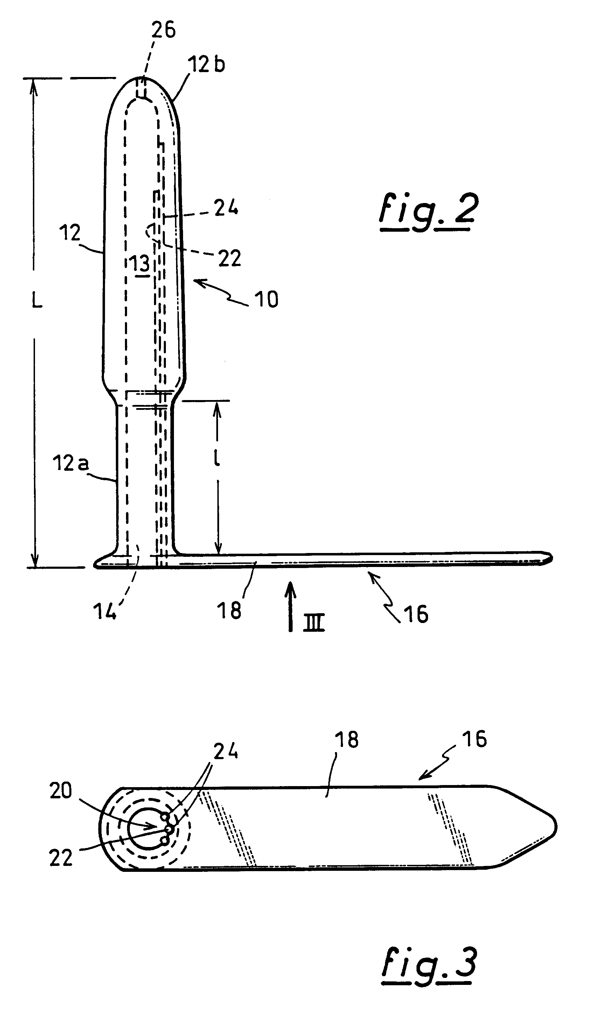

Selon une autre caractéristique avantageuse de l'invention, le corps 12 de la sonde rectale est pourvu à son extrémité voisine de l'ouverture 14, d'un moyen d'arrêt d'insertion 16 de la sonde rectale 10 dans le rectum R. De préférence, ce moyen d'arrêt 16 comprend une languette 18 s'étendant extérieurement radialement par rapport au corps de la sonde 12, de préférence dans un plan sensiblement perpendiculaire au corps de la sonde, ce qui est encore bien visible à la figure 2.

En raison de la souplesse du matériau polymère constituant la sonde 12 et aussi la languette 18, cette languette se déforme lors de l'insertion pour épouser parfaitement le corps du patient comme cela est clairement visible à la figure 1 ce qui assure un positionnement anatomique du dispositif formant sonde rectale 10.

Selon une autre caractéristique avantageuse de l'invention, la longueur total L du corps de la sonde 12 est choisie pour permettre l'insertion d'un instrument au moins en regard d'un organe à observer ou traiter, en particulier en regard de la prostate, en position insérée dans le rectum, ce qui est encore visible à la figure 1.

Selon une autre caractéristique avantageuse de l'invention, le corps de la sonde 12 comprend sur sa paroi interne et/ou externe au moins un dispositif 20 capteur de température. De préférence, ce dispositif capteur de température est disposé du même côté que le moyen d'arrêt 16, c'est-à-dire la languette 18, ce qui facilite le positionnement en regard de l'organe à observer, ou traiter, en particulier de la prostate P.

Ce dispositif capteur de température comprend avantageusement au moins un thermomètre à fibres optiques 22 logées dans une rainure correspondante de la paroi, de préférence interne, du corps 12 de la sonde. Avantageusement, ce thermomètre comprend plusieurs fibres optiques disposées décalées radialement et/ou longitudinalement dans le sens longitudinal du corps 12 de sonde, de manière à détecter la température sur diverses positions radiales et/ou longitudinales de la sonde.

Le dispositif formant sonde rectale 10 peut également comprendre des moyens de mesure de champ électrique, permettant de mesurer le champ électrique émis par un dispositif générateur de champ électrique 30, par exemple introduit dans l'urètre U jusque dans la prostate P, pour réaliser un traitement thérapeutique de la prostate P. Un dispositif 30 peut être constitué par une sonde émettrice du déposant, en particulier FR-8815126, FR-9001478 et FR-900321. Un tel dispositif comprend un ballonnet 32 de positionnement introduit jusque dans la vessie V.

Ainsi, la présente invention couvre également un appareil de traitement thérapeutique caractérisé en ce qu'il comprend une sonde rectale 10 telle que précédemment définie.

On comprend que grâce à l'invention, il est possible d'introduire un instrument de détection de champ électrique ou de température, dans la sonde rectale 10, en vue d'observer ou de traiter un organe à observer ou à traiter disposé au voisinage du rectum R, par exemple la prostate P, d'une manière précise, sûre et fiable. Le dispositif formant sonde rectale 10 selon l'invention, équipé des moyens de mesure de champ électrique ou de température tel que les moyens 20, 22, permet en outre de mesurer le champ électrique ou la température au niveau du rectum R, ce qui améliore de manière significative la surveillance du traitement thérapeutique d'un organe à traiter, en particulier de la prostate.

Il est à noter que le dispositif formant sonde rectale tel que représenté aux figures 1 à 3 fait partie intégrante de l'invention et donc partie intégrante de la présente description.

## Revendications

1. Dispositif formant sonde rectale (10), caractérisé en ce qu'il comprend un corps de sonde (12) réalisé en un matériau polymère autoportant souple dont le degré de souplesse est défini par la dureté Shore A, qui est inférieure à environ 90, pour épouser la forme du rectum (R), tout en ne réalisant sensiblement pas d'effet de compression sur le rectum (R), lorsqu'il est introduit dans le rectum ; et en ce que le corps de sonde (12) comprend :
a) des moyens de mesure d'un champ électrique, ou
b) au moins un dispositif capteur de température, les moyens de mesure ou le dispositif étant disposés en combinaison avec un moyen d'arrêt (16) pour faciliter le positionnement du dispositif capteur de température ou des moyens de mesure d'un champ électrique en regard d'un organe à observer ou à traiter, en particulier en regard de la prostate.

2. Dispositif selon la revendication 1, caractérisé en ce que le matériau polymère précité a une dureté Shore A qui est comprise entre 20 et 90, en particulier entre 60 et 80.

3. Dispositif selon la revendication 1 ou 2, caractérisé en ce que le corps de sonde (12) comprend un rétrécissement (12a) de diamètre, en regard du sphincter (S), ce rétrécissement (12a) favorisant un maintien du corps de sonde (12) en place par le sphincter (S), de préférence la longueur (l) du rétrécissement (12a) correspondant sensiblement à la dimension du sphincter (S).

4. Dispositif selon l'une des revendications 1 à 3, caractérisé en ce que l'épaisseur du corps de sonde (12) varie entre environ 1 et environ 3,5 mm selon la dimension.

5. Dispositif selon l'une des revendications 1 à 4, caractérisé en ce que le corps de sonde (12) est réalisé en un matériau polymère présentant une dureté Shore A comprise entre 60 et 80, et est de préférence choisi parmi un élastomère tel qu'un caoutchouc, un polyuréthanne ; ou une matière plastique telle que le polyéthylène ou du PVC.

6. Dispositif selon l'une des revendications 1 à 5, caractérisé en ce que le corps de sonde (12) est pourvu d'un moyen (16) d'arrêt d'insertion du dispositif formant sonde (10) dans le rectum (R), de préférence réalisé avec le même matériau que le corps de sonde (12).

7. Dispositif selon la revendication 6, caractérisé en ce que le moyen d'arrêt (16) comprend une languette (18) s'étendant extérieurement radialement par rapport au corps (12) de la sonde, de préférence dans un plan sensiblement perpendiculaire au corps (12) de la sonde.

8. Dispositif selon l'une des revendications 1 à 7, caractérisé en ce que la longueur (L) du corps (12) de la solide rectale (10) permet d'aller au moins en regard d'un organe à observer, ou à traiter, en particulier de la prostate, en position insérée dans le rectum pour permettre de capter la température par le dispositif (20) capteur de température précité, ou de mesurer un champ électrique par les moyens de mesure d'un champ électrique précité.

9. Dispositif selon la revendication 8, caractérisé en ce que le corps de sonde (12) précité est creux tout en étant sensiblement fermé à son extrémité avant et ouvert à son extrémité arrière ; avantageusement, il comprend une ouverture (14) permettant l'insertion d'un instrument de détection ou de repérage d'un organe à traiter, en particulier de la prostate, de préférence comprenant une sonde échographique, en particulier de type ultrasonique permettant de visualiser l'organe à traiter, en particulier la prostate.

10. Dispositif selon l'une des revendications 1 à 9, caractérisé en ce que le dispositif capteur de température ou les moyens de mesure de champ électrique est/sont disposé(s) du même côté que les moyens d'arrêt d'insertion (16), de manière à faciliter le positionnement en regard de l'organe à observer, en particulier de la prostate (P).

11. Dispositif selon la revendication 9 ou 10, caractérisé en ce que le dispositif capteur de température précité (20) comprend au moins un thermomètre à fibres optiques (22) logées dans une ou plusieures rainures de la paroi, de préférence interne, du corps (12) de la sonde, avantageusement ce thermomètre comprend plusieurs fibres optiques disposées décalées radialement et/ou longitudinalement dans le sens longitudinal du corps (12) de la sonde de manière à détecter la température sur diverses positions radiales et/ou longitudinales de la sonde.

12. Dispositif selon l'une quelconque des revendications 1 à 11, caractérisé en ce que le corps de sonde (12) comprend à son extrémité avant ou interne (12b) un orifice (26) permettant l'évacuation des gaz éventuellement contenus dans le rectum.

13. Appareil de traitement thérapeutique, caractérisé en ce qu'il comprend un dispositif formant sonde rectale (10) tel que défini à l'une quelconque des revendications 1 à 12.

14. Appareil de traitement thérapeutique selon la revendication 13, caractérisé en ce qu'il comprend des moyens (30) de traitement thérapeutique de tissus, en particulier par hyperthermie, de préférence de traitement thérapeutique de la prostate (P).

15. Appareil de traitement thérapeutique selon la revendication 14, caractérisé en ce que les moyens (30) de traitement thérapeutique de tissus comprennent un dispositif générateur de champ électrique (30) introduit dans l'urètre U jusque dans la prostate (P) pour réaliser un traitement thérapeutique de la prostate (P), par exemple comprenant une sonde émettrice à micro-ondes comprenant un ballonnet (32) de positionnement introduit jusque dans la vessie (V).

## Claims

1. A device constituting a rectal probe (10), characterized in that it comprises a probe body (12) made of a flexible self-supporting polymer material whose degree of flexibility is defined by a Shore A hardness of less than about 90, to enable it to comply with the shape of the rectum (R) while having substantially no compression effect on the rectum (R) when inserted therein; and in that the probe body (12) comprises:
a) electric field measuring means, or
b) at least one temperature sensor device, the measuring means or the device being disposed in combination with stop means (16) in order to facilitate positioning of the temperature sensor device or of the electric field measuring means level with an organ to be observed or treated, in particular level with the prostate.

2. The device according to claim 1, characterized in that the above-mentioned polymer material has a Shore A hardness lying in the range 20 to 90, and in particular in the range 60 to 80.

3. The device according to claim 1 or 2, characterized in that the probe body (12) includes a neck portion (12a) of reduced diameter and coming level with the sphincter (S), said neck portion (12a) facilitating holding of the probe body (12) in place by the sphincter (S), preferably the length (l) of the neck portion (12a) substantially corresponding to the size of the sphincter (S).

4. The device according to one of claims 1 to 3, characterized in that the thickness of the probe body (12) varies between about 1 and about 3.5 mm depending on the size.

5. The device according to one of claims 1 to 4, characterized in that the probe body (12) is made of a polymer material having a Shore A hardness lying in the range 60 to 80, and is preferably selected from an elastomer such as a rubber, a polyurethane, or a plastics material such as polyethylene or PVC.

6. The device according to one of claims 1 to 5, characterized in that the probe body (12) is provided with insertion stop means (16) for stopping insertion of the probe-forming device (10) into the rectum (R), preferably made of the same material as the probe body (12).

7. The device according to calm 6, characterized in that the stop means (16) comprises a tongue (18) extending radially outwards from the probe body (12), preferably in a plane which is substantially perpendicular to the probe body (12).

8. The device according to one of claims 1 to 7, characterized in that the length (L) of the body (12) of the rectal probe (10) makes it possible to come at least level with an organ to be observed or treated, in particular the prostate, when the probe is in its inserted position in the rectum, so as to sense the temperature by the aforesaid temperature sensor device (20) or to measure an electric field by the aforesaid electric field measuring means.

9. The device according to calm 8, characterized in that the above-mentioned probe body (12) is hollow, while being substantially closed at its front end and open at its rear end; advantageously it includes an opening (14) enabling the insertion of an instrument for detecting or locating an organ to be treated, in particular the prostate, preferably comprising an echographic probe, in particular of ultrasonic type enabling to display the organ to be treated, in particular the prostate.

10. The device according to one of claims 1 to 9, characterized in that the temperature sensor device or the electric field measuring means are disposed on the same side as the insertion stop means (16), thereby facilitating positioning level with the organ to be observed, in particular the prostate (P).

11. The device according to claim 9 or 10, characterized in that the above-mentioned temperature sensor device (20) comprises at least one optical fiber thermometer (22) received in one or more grooves in the wall, and preferably the inside wall, of the probe body (12), this thermometer advantageously comprises a plurality of optical fibers that are arranged so as to be offset radially and/or longitudinally in the longitudinal direction of the probe body (12) in order to detect the temperature over various radial and/or longitudinal positions of the probe.

12. The device according to any one of claims 1 to 11, characterized in that the probe body (12) includes an orifice (26) in its front or inside end (12b) for evacuating gasses that may be contained in the rectum.

13. A therapeutic treatment apparatus, characterized in that it includes a device constituting a rectal probe (10) as defined in any one of claims 1 to 12.

14. The therapeutic treatment apparatus according to claim 13, characterized in that it includes means (30) for the therapeutic treatment of tissues, in particular by hyperthermia, and preferably for the therapeutic treatment of the prostate (P).

15. The therapeutic treatment apparatus according to claim 14, characterized in that the means (30) for the therapeutic treatment of tissues comprise an electric field generator device (30) introduced in the urethra (U) into the prostate (P) in order to perform the therapeutic treatment of the prostate (P), for example comprising a microwave-emitting probe comprising a positioning balloon (32) which is inserted into the bladder (V).

## Patentansprüche

1. Eine Rektalsonde (10) bildende Vorrichtung, dadurch gekennzeichnet, daß sie einen Sondenkörper (12) aus einem formhaltigen, nachgiebigen Polymermaterial umfaßt, dessen Nachgiebigkeitsgrad durch die Shore-Härte A, die unter etwa 90 liegt, definiert ist, zur Annahme der Form des Rektums (R), gleichzeitig aber beim Einführen in das Rektum im wesentlichen keinen Druck auf das Rektum (R) ausübt; und daß der Sondenkörper (12) aufweist:
a) Mittel zur Messung eines elektrischen Feldes, oder
b) mindestens einen Temperaturfühler, wobei die Mittel zur Messung oder die Vorrichtung in Kombination mit einem Mittel zum Anhalten (16) vorliegen, um die Positionierung des Temperaturfühlers oder der Mittel zur Messung eines elektrischen Feldes in bezug auf ein zu beobachtendes oder zu behandelndes Organ, insbesondere die Prostata, zu erleichtern.

2. Vorrichtung nach Anspruch 1, dadurch gekennzeichnet, daß das Polymermaterial eine Shore-Härte A aufweist, die zwischen 20 und 90, insbesondere zwischen 60 und 80, liegt.

3. Vorrichtung nach Anspruch 1 oder 2, dadurch gekennzeichnet, daß der Sondenkörper (12) eine Durchmesserverengung (12a) im Bereich des Sphinkters (S) aufweist, welche Verengung (12a) die Halterung des Sondenkörpers (12) in seiner Lage durch den Sphinkter begünstigt, wobei die Lange (l) der Verengung (12a) vorzugsweise im wesentlichen der Abmessung des Sphinkters (S) entspricht.

4. Vorrichtung nach einem der Ansprüche 1 bis 3, dadurch gekennzeichnet, daß die Dicke des Sondenkörpers (12) dimensionsgemäß zwischen ca. 1 und ca. 3,5 mm variiert.

5. Vorrichtung nach einem der Ansprüche 1 bis 4, dadurch gekennzeichnet, daß der Sondenkörper (12) aus einem Polymermaterial hergestellt ist, das eine Shore-Harte A zwischen 60 und 80 aufweist und vorzugsweise ausgewählt ist aus einem Elastomer, wie einem Kautschuk, einem Polyurethan; oder einem Kunststoff, wie Polyethylen oder PVC.

6. Vorrichtung nach einem der Ansprüche 1 bis 5, dadurch gekennzeichnet, daß der Sondenkörper (12) mit einem Mittel (16) zum Anhalten der Einführung der die Sonde (10) bildenden Vorrichtung in das Rektum (R) versehen ist, das vorzugsweise aus demselben Material wie der Sondenköper (12) gebildet ist.

7. Vorrichtung nach Anspruch 6, dadurch gekennzeichnet, daß das Mittel (16) zum Anhalten eine Zunge (18) aufweist, die sich außen radial in bezug auf den Sondenkörper (12), vorzugsweise in einer zum Sondenkörper (12) im wesentlichen senkrechten Ebene, erstreckt.

8. Vorrichtung nach einem der Ansprüche 1 bis 7, dadurch gekennzeichnet, daß die Länge (L) des Köpers (12) der Rektalsonde (10) einen Verlauf bis mindestens zu dem Bereich eines zu beobachtenden oder zu behandelnden Organs, insbesondere der Prostata, in der im Rektum eingeführten Position ermöglicht, um ein Abfühlen der Temperatur durch den Temperaturfühler (20) oder eine Messung eines elektrischen Feldes durch die Mittel zur Messung eines elektrischen Feldes zu gestatten.

9. Vorrichtung nach Anspruch 8, dadurch gekennzeichnet, daß der Sondenkörper (12) hohl, dabei aber an seinem vorderen Ende im wesentlichen geschlossen und an seinem hinderen Ende offen ist, wobei er vorteilhaft eine Öffnung (14) besitzt, die das Einführen eines Instruments zur Detektion oder zur Ortung eines zu behandelnden Organs, insbesondere der Prostata, ermöglicht, vorzugsweise mit einer Echogrammsonde, insbesondere vom Ultraschalltyp, welche eine Sichtbarmachung eines zu behandelnden Organs, insbesondere der Prostata (P), ermöglicht.

10. Vorrichtung nach einem der Ansprüche 1 bis 9, dadurch gekennzeichnet, daß der Temperaturfühler oder die Mittel zur Messung des elektrischen Feldes auf derselben Seite wie die Mittel (16) zum Anhalten der Einführung angeordnet ist/sind, um die Positionierung gegenüber dem zu beobachtenden Organ, insbesondere der Prostata (P), zu erleichtern.

11. Vorrichtung nach Anspruch 9 oder 10, dadurch gekennzeichnet, daß der Temperaturfühler (20) mindestens ein Lichtleiter-Thermometer (22) aufweist, das in einer oder mehreren Rillen der Wand, vorzugsweise der Innenwand, des Köpers (12) der Sonde angeordnet ist, wobei das Thermometer vorteilhaft mehrere Lichtleiter aufweist, die radial und/oder der Länge nach in der Längsrichtung des Körpers (12) der Sonde verschoben angeordnet sind, um die Temperatur über verschiedenen Radial- und/oder Längspositionen der Sonde zu detektieren.

12. Vorrichtung nach einem der Ansprüche 1 bis 11, dadurch gekennzeichnet, daß der Sondenkörper (12) an seinem vorderen oder inneren Ende (12b) eine Öffnung (26) aufweist, die die Entfernung eventuell im Rektum vorhandener Gase ermöglicht.

13. Therapeutischer Behandlungsapparat, dadurch gekennzeichnet, daß er eine eine Rektalsonde (10) bildende Vorrichtung nach einem der Ansprüche 1 bis 12 aufweist.

14. Therapeutischer Behandlungsapparat nach Anspruch 13, dadurch gekennzeichnet, daß er Mittel (30) zur therapeutischen Behandlung von Geweben, insbesondere durch Hyperthermie, vorzugsweise zur therapeutischen Behandlung der Prostata (P), aufweist.

15. Therapeutischer Behandlungsapparat nach Anspruch 14, dadurch gekennzeichnet, daß die Mittel (30) zur therapeutischen Behandlung von Geweben eine in die Harnröhre U bis zur Prostata (P) eingeführte Erzeugungseinrichtung für ein elektrisches Feld (30) aufweisen, um eine therapeutische Behandlung der Prostata (P), beispielsweise mit einer Mikrowellen emittierenden Sonde mit einem Positionierungs-Ballonett (32), das bis in die Blase (V) eingeführt ist, durchzuführen.
